# EUROPEAN PATENT APPLICATION

(11) **EP 4 821 432 A1**
(43) Date of publication of application: **14.10.2026**
(21) Application number: 25169496.4
(22) Date of filing: 09.04.2025
(51) Int. Cl.: A61B 5/11, A61B 5/00

(54) **SYSTEM AND METHOD FOR MONITORING WALKING SPEED OF SUBJECT**

(71) Applicant: Koninklijke Philips N.V., 5656 AE Eindhoven (NL)
(72) Inventor: FEDJAJEVS, Andrejs, 5656 AG Eindhoven (NL); COX, Lieke Gertruda Elisabeth, 5656 AG Eindhoven (NL); PAPINI, Gabriele, 5656 AG Eindhoven (NL); MÜSCH, Guido Josef, 5656 AG Eindhoven (NL)
(74) Representative: Philips Intellectual Property & Standards

(57) **Abstract**

A method of monitoring walking speed of a subject includes estimating a reference walking speed of the subject based on walking pattern variations in a test-specific pattern of a walking test over a known distance; predicting a calibration estimated walking speed of the subject using a predetermined walking speed algorithm based on data from at least one sensor worn by the subject during the walking test; determining at least one calibration coefficient based on the reference walking speed and the calibration estimated walking speed; predicting a subsequent estimated walking speed of the subject during a subsequent walking event after the walking test; and determining a corrected walking speed of the subject during the subsequent walking event by adjusting the subsequent estimated walking speed using the at least one calibration coefficient.

## Description

### FIELD OF THE INVENTION

The invention relates to the field of medical monitoring and diagnosis, and more specifically to providing corrected walking speed estimations.

### BACKGROUND OF THE INVENTION

Activity metrics are important to assess the medical condition or overall well-being of a subject. Continuous and remote monitoring of activity and cardiorespiratory metrics has gained popularity as a means to enhance patient care, reduce hospital stays, and improve patient outcomes. These metrics can be used to track recovery or deterioration trends of a subject over extended periods of time. Walking speed, in particular, is one of such activity metric, which is a strong indicator of the subject's state or recovery trend, e.g., following an illness or medical procedure. For example, an increase in walking speed, whether during standardized activity tests or free-living conditions, may indicate a trend toward recovery.

Subjects are typically monitored for extended periods of time while performing various activities, with and without supervision from healthcare professionals, including walking speed. To obtain reliable data, walking speed is monitored in a structured manner, such as during a conventional standardized two-minute-walking-test (2MWT) or six-minute-walking-test (6MWT), as well as during free-living or treadmill walking. During the 6MWT, in particular, the subject is asked to walk a straight line for a distance of 30 meters as many times as possible within six minutes. At the end of the 30 meter distance, the subject turns around and continues moving in the opposite direction for another 30 meters. The 6MWT is a simple, low-cost test that can be performed in a variety of settings, including hospitals, rehabilitation centers, and even in the subject's home. The results of the 6MWT may be used to evaluate effectiveness of various interventions, such as medications or rehabilitation programs, in improving the subject's physical functioning and overall health. For example, for a subject with heart failure, a shorter distance walked during the 6MWT is associated with deteriorating conditions, with a higher risk of hospitalization and mortality. However, standardized walking tests require clinical staff to be present, which is time-consuming and expensive. Further, standardized walking tests are executed only at certain time intervals with the patients walking, typically back and forth along predetermined distances.

Further, walking speed may be estimated from signals output be a sensor worn by the subject, such as an accelerometer, using specialized walking algorithms. However, conventional walking algorithms used to estimate walking speed based on accelerometer signals are susceptible to errors. The errors may arise from various sources, including measurement noise, training data imbalance (i.e., uneven representation of various speed samples), sensor placement bias, and differences in physique and gait patterns among individuals, for example. Some of these error sources are personalized and may persist over extended periods of measurement, even across different activities or changes in activity.

### SUMMARY OF THE INVENTION

According to a representative embodiment, a method is provided for monitoring walking speed of a subject. The method includes estimating a reference walking speed of the subject based on walking pattern variations in a test-specific pattern of a walking test over a known distance; predicting a calibration estimated walking speed of the subject using a predetermined walking speed algorithm based on data from at least one sensor worn by the subject during the walking test; determining at least one calibration coefficient based on the reference walking speed and the calibration estimated walking speed; predicting a subsequent estimated walking speed of the subject during a subsequent walking event using the predetermined walking speed algorithm after the walking test; and determining a corrected walking speed of the subject during the subsequent walking event by adjusting the subsequent estimated walking speed using the at least one calibration coefficient.

According to another representative embodiment, a system is provided for monitoring walking speed of a subject. The system includes at least one sensor worn by the subject, at least one processor configured to receive data from the at least one sensor, and at least one non-transitory memory. The memory stores instructions that, when executed by the at least one processor to, cause the at least one processor to estimate a reference walking speed of the subject based on walking pattern variations in a test-specific pattern of a walking test over a known distance; predict a calibration estimated walking speed of the subject using a predetermined walking speed algorithm based on data from at least one sensor during the walking test; determine at least one calibration coefficient based on the reference walking speed and the calibration estimated walking speed; predict a subsequent estimated walking speed of the subject during a subsequent walking event using the predetermined walking speed algorithm after the walking test; and determine a corrected walking speed of the subject during the subsequent walking event by adjusting the subsequent estimated walking speed using the at least one calibration coefficient.

According to another representative embodiment, a computer program product is provided causing the system as disclosed herein to execute the steps of the method as disclosed herein. Preferred embodiments of the invention are defined in the dependent claims. It shall be understood that the claimed method, system and computer program product have similar and/or identical preferred embodiments, in particular as defined in the dependent claims and as disclosed herein.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 is a simplified block diagram of a system for monitoring walking speed of a subject, according to a representative embodiment.
FIG. 2 is a flow diagram of a method of monitoring walking speed of a subject, according to a representative embodiment.
FIG. 3 is a graph showing reference walking speeds and calibration estimated walking speeds, according to a representative embodiment, where the calibration estimated walking speeds are predicted using on four different walking speed algorithms.
FIG. 4 is a graph showing a known walking speed, subsequent estimated walking speeds predicted using the four different walking speed algorithms, and corrected walking speeds determined during a subsequent walking event, according to a representative embodiment.

### DETAILED DESCRIPTION OF THE EMBODIMENTS

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings.

Generally, the various embodiments described herein provide a system and method for correcting estimated speed values using information from previously executed walking tests that include walking pattern variations, such as turning events and/or body position transitions. For example, during standardized walking test that includes one or more turning events by a subject, the turning events are automatically identified and used to correct walking speed estimation by existing or yet to be determined walking speed algorithms within the purview of one of ordinary skill in the art having the benefit of the present disclosure. In particular, the various embodiments include calculating a calibration coefficient as the ratio between a turn-based walking speed estimation and a calibration estimated walking speed from the walking speed algorithm, and applying the calibration coefficient to the same walking speed algorithm used to estimate walking speed of the subject in a subsequent walking event. Accordingly, the subsequent walking event may be performed without supervision by clinical staff, enabling the subject to assess recovery more continuously, i.e., without the clinical staff being present. Also, correcting the walking speed estimations using the calibration coefficient provides more reliable results and better confidence intervals.

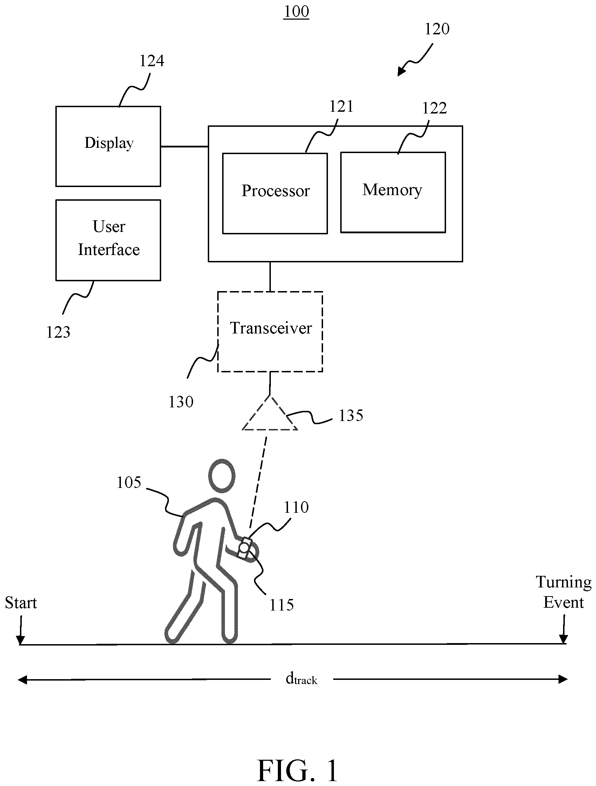

FIG. 1 is a simplified block diagram a system for monitoring walking speed of a subject, according to a representative embodiment.

Referring to FIG. 1, the system 100 includes a monitor device 110 that is worn by subject 105 and configured to monitor walking speed of the subject 105. The walking speed is monitored during a walking test for calibration and during a subsequent walking event, during which the walking speed is corrected according to the calibration. The walking test is indicated by a Start position and a Turning Event position that is a known distance or segment from the Start position, indicated by track distance (d_{track}). The Turning Event is an example of a walking pattern variation in a test-specific pattern of the walking test, since the subject 105 must slow down entering the Turning Event, perform a 180 degree change of direction, and speed up exiting the Turning Event, as discussed below. Other examples of walking pattern variations include body position transitions of the subject 105, such as moving from sitting to standing positions at the beginning of the walking test and/or moving from standing to sitting positions at the end of the walking test, changes in body posture of the subject 105, and starting and stopping walking by the subject 105.

In various embodiments, the walking test may be a standardized walking test, such as six-minute walking test (6MWT), a two-minute walking test (2MWT), or a timed up and go (TUG) walking test, for example. Each of the standardized walking tests has a test-specific pattern that includes one or more turning events that are spaced apart by the known distance. For example, the known distance of the track each in each of the 6MWT and the 2MWT is 30 meters between turning events. The subject 105 repeats traversing the track and performing 180 degree turns for six minutes or for two minutes, respectively. In the TUG walking test, for example, the known distance of the track is 3 meters. The TUG walking test includes the subject 105 initially standing at the Start position, traversing the track, performing a 180 turn at the Turning Event, returning along the track, and sitting at the Start position. Alternatively, the walking test may be a customized walking test that has a similar structure to one or more known walking tests (i.e., walking pattern variation(s) plus fixed walking distance(s)). For example, a user may define a new walking test that consists of the subject 105 standing, walking 50 meters, turning, walking 50 meters, and sitting. Of course, any walking test with a known test-specific pattern, including known walking pattern variations and known distances between the walking pattern variations, without departing from the scope of the present teachings.

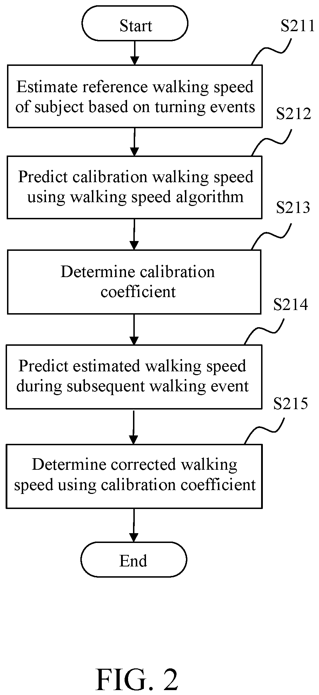

The monitor device 110 includes a wearable sensor 115 configured to detect movement of the subject 105. The sensor 115 may be any compatible inertial, optical, or angular sensor capable of collecting movement data for measuring bodily movement of the subject 105, such as an accelerometer, a gyroscope, a barometer, or a force sensor (e.g., in insoles), for example, as further described below with reference to FIG. 2. The sensor 115 is wearable in that it is located on and/or attached to the body of the subject 105. In this regard, although the sensor 115 is shown on the wrist of the subject 105, it is understood that the sensor 115 may be located anywhere of the subject's body where movement may be determined, such as the ankle, knee, arm or torso of the subject 105, for example, as would be apparent to one skilled in the art. Also, it is understood that the sensor 115 is representative of one or more sensors that may be used for providing different types of movement data for measuring movement of the subject 105. For example, the sensor 115 may be implemented as a first sensor (e.g., barometer) for detecting when the subject 105 initially stands up at the Start of the walking test and a second sensor (e.g., accelerometer) for detecting when the subject 105 slows down and speeds up when entering and exiting the Turning Event, respectively, which occurs a known distance from the Start of the walking test. It is further understood that non-wearable sensors may be used instead of or in addition to the sensor 115. For example, the walking speed of the subject 105 may be estimated using a movement sensor, such as a motion sensor or a video camera, that is positioned apart from the subject 105 in order to observe and collect movement data indicating the walking pattern and walking pattern variations of the subject 105 during the walking test.

The system 100 further includes a processing unit 120 configured to receive, process and store the measurement data collected by the sensor 115. In the depicted embodiment, the processing unit 120 is located remotely from the monitor device 110, in which case the system 100 optionally includes a transceiver 130 and an antenna configured to receive the measurement data from the monitor device 110 and to transmit control signals to the monitor device 110 over a wireless radio frequency (RF) communication network, as would be apparent to one skilled in the art. In this case, the monitor device 110 would likewise include a transceiver and antenna (not shown). In an alternative embodiment, all or part of the functionality of the processing unit 120 may be provided with the monitor device 110 without departing from the scope of the present teachings. The processing unit 120 is configured to perform walking speed calibration to determine a calibration coefficient specific to the subject and the walking speed algorithm used for calibration, and to perform subsequent walking speed estimation of the subject 105 corrected for inaccuracies in the walking speed algorithm using the calibration coefficient, as discussed below with reference to FIG. 2.

The processing unit 120 includes one or more processors indicated by processor 121 and one or more memories indicated by memory 122. The processing unit 120 may further include a user interface 123 and a display 124, discussed below. The processor 121 communicates with the monitor device 110 through a monitor device interface (not shown), as well as the transceiver 130 and the antenna 135, discussed above, as would be apparent to one skilled in the art. The processor 121 may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hardwired logic circuits, or combinations thereof. The processor 121 may include multiple processors, parallel processors, or both. The processor 121 may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 122 stores instructions executable by the processor 121 that, when executed, cause the processor 121 to implement all or part of various more processes for monitoring walking speed, include the method described with reference to FIG. 2, below. The memory 122 may include main memory and/or static memory, where such memories may communicate with each other and the processor 121 via one or more buses. The memory 122 may be implemented by any number, type and combination of random access memory (RAM) and read-only memory (ROM), for example, and may store various types of information, such as software algorithms, artificial intelligence (AI) machine learning models, and computer programs, all of which are executable by the processor 121. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 122 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 122 may store software instructions and/or computer readable code that enable performance of various functions. The memory 122 may be secure and/or encrypted, or unsecure and/or unencrypted.

The user interface 123 is configured to provide information and data output by the processor 121, and the memory 122, and/or the monitoring device 110 to the user and/or for receiving information and data input by the user. That is, the user interface 123 enables the user to enter data and to control or manipulate aspects of the processes described herein, and also enables the processor 121 to indicate the effects of the user's input. All or a portion of the user interface 123 may be implemented by a graphical user interface (GUI). The user interface 123 may include one or more interface devices, such as a touchpad, a touchscreen, a mouse, a keyboard, a trackball, a joystick, or a microphone, for example.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes a screen for viewing information with regard to walking speed. When the processing unit 120 is located remotely from the monitor device 110, the user interface 123 and the display 124 may be located at a workstation with the processor 121 and the memory 122 to enable interfacing with the user, e.g., observing the walking test or the subsequent walking event. Alternatively, when the processing unit 120 is located with the monitor device 110, and thus worn by the user/subject 105, the user interface 12 and the display 124 also may be located on the monitor device 110 with the processor 121 and the memory 122.

FIG. 2 is a flow diagram of a method of monitoring walking speed of a subject (e.g., subject 105), according to a representative embodiment. The method may be implemented at least in part using instructions stored in memory 122 and executable by the processor 121 in the processing unit 120, for example, discussed above.

Referring to FIG. 2, the method of monitoring walking speed of the subject includes estimating a reference walking speed of the subject in a walking test in block S211. In various embodiments, the reference walking speed is the average walking speed of the subject, which is estimated during at least a portion of the walking test. The walking test may be a standardized walking test, such as a 6MWT, a 2MWT, or a TUG walking test, for example, discussed above. The walking test has a test-specific pattern that includes walking pattern variations, such as turning events, for example, which are spaced apart by a known distance. For example, each of the 6MWT and the 2MWT has a known distance of 30 meters between turning events. Each turning event involves the subject performing a 180 degree turn after traversing a straight path (track) of the known distance in order to return along the same straight path in the opposite direction. Other walking pattern variations include body position transitions, changes in body posture, and starting and stopping walking by the subject, as discussed above.

Generally, when using turning events, the reference walking speed is estimated by detecting the turning events in the test-specific pattern during the walking test based on a variation in the subject's walking pattern, and determining the elapsed time between consecutive turning events (or other walking pattern variations). For example, according to the 6MWT, the standing subject starts to walk (first walking pattern variation), walks 30 meters straight, turns 180 degrees (second walking pattern variation), walks 30 meters straight in the opposite direction, turns 180 degrees (third walking pattern variation), repeats until six minutes has elapsed from the first walking pattern variation, and stops walking (nth walking pattern variation). The 2MWT is the same, except the subject repeats the test-specific pattern until two minutes has elapsed from the first walking pattern variation. According to the TUG walking test, the subject stands up and starts walking (first walking pattern variation), walks three meters, turns 180 degrees (second walking pattern variation), walks back three meters, and stops walking and sits down (third walking pattern variation). Estimating speed based on turning events, as opposed to simply calculating distance over time as in conventional walking tests, uses time and distance values, but is able to provide more fine-grained outputs specific to segments of the walking pattern between the walking pattern variations.

For example, turning events may be detected using a turn detection algorithm based on the calibration estimated walking speed of the subject predicted by a predetermined walking speed algorithm, discussed below. The turn detection algorithm may determine that the subject is approaching a turn when the estimated walking speed decreases and is exiting the turn when the estimated walking speed subsequently increases. The turn detection may be refined by considering the rate of speed change and regularity of occurrence of the speed change. For example, in the 6MWT, it may be assumed that the turning events regularly occur about the same interval time apart, so changes in the estimated walking speed occurring closer together may be ignored or further apart. From the reference distance, it may be deduced how many turns should be detected, and how far in time they are on average apart, so that outliers may be detected. Notably, the 6MWT distance (or other test outcome in case of another standardized walking test) may be measured by the user conducting the test and fed back, e.g., using the user interface 123, to check whether any turns of the 6MWT were missed or wrongly detected by the turn detection algorithm.

The walking pattern variations of the subject are detected as movement data using one or more sensors (e.g., sensor 115). The movement data from the sensors are provided to a processing unit (e.g., processing unit 120), which is configured to identify each of the walking pattern variations (e.g., turning events, changes in position), e.g., using a turn detection algorithm using sensor data, as discussed below, and to determine elapsed time between consecutive walking pattern variations over a known distance.

As mentioned above, different types of wearable sensors include an accelerometer, a gyroscope, a barometer, and a force sensor, for example. An accelerator detects increases and decreases in the subject's speed during the walking test, as well as changes in the subject's orientation and general changes in the movement pattern. Therefore, using an accelerometer as the sensor, the walking pattern variations (e.g., starting, stopping and turning events) may be identified by sudden and momentary increases and decreases in the estimated speed. For example, the subject slows down and stops in the horizontal direction to make a turn, and then speeds up to walking speed after completing the turn. The accelerometer may also detect a specific acceleration signal pattern, such as decreased amplitude during a turn or a typical posture transition pattern, which may be used to determine turning events.

More particularly, a processor (e.g., processing unit 120 and/or a processor in the accelerometer sensor) may execute a turn detection algorithm that identifies a turn based on signals from the accelerometer, as would be apparent to one skilled in the art. For example, for a three axial accelerometer, turns are detected based on a decrease of amplitude of the accelerometer signal on the axis parallel to gravity followed by an increase of amplitude, indicating the subject slowing down and speeding up before and after the turn. Starting is detected based on signals with no amplitude followed by signals with some amplitude indicating movement, while stopping is detected based on signals with some amplitude followed by data signals with no amplitude indicating lack of movement,

A gyroscope detects orientation and angular velocity of the subject during the walking test. Therefore, using a gyroscope, the walking pattern variations may be determined based on changes in the subject's orientation and angular velocity. For example, integrating the angular velocity detected by the gyroscope, it is possible to estimate the turning angle. Therefore, a turning event may be identified as an angular velocity change between N and M degrees during a turn, where N and M depend on the type of turn. For example, when the subject turns 180 degrees, integrating the output of the gyroscope may provide a change between about 170 degrees and about 190 degrees.

Again, a processor (e.g., processing unit 120 and/or a processor in the gyroscope sensor) may execute a turn detection algorithm that identifies a turn based on signals from the gyroscope, as would be apparent to one skilled in the art. For example, using a gyroscope mounted to the subject's chest, turns are detected based on direct calculation of the turning angel, mentioned above. Standing and sitting are detected based on gyroscope signals indicating a transitory forward bending movement (from an angle calculation).

A barometer detects atmospheric pressure. Therefore, using a barometer, walking pattern variations involving vertical movement of the subject, such as standing up and sitting down, e.g., at the beginning and the end of the walking test, may be determined based on decreases and increases in atmospheric pressure.

A force sensor detects force or impact exerted on the subject's body. For example, force sensors may be included in the insoles of the subjects' shoes, where they detect the impact of the subject's feet hitting the ground. This enables the force sensor to detected the presence and number of steps performed by the subject.

As mentioned above, the reference walking speed may be the average walking speed of the subject as determined during the walking test based on turning events. The average walking speed may be determined using all or a portion of the walking test. For example, in an embodiment, the average walking speed is determined based on the cumulative distance walked by the subject between all of the turns and the corresponding time taken to walk to the cumulative distance, which may be calculated according to Equation (1): $Average Speed = \left(number of turns*{d}_{track}\right) / \left(time of last turn-test start time\right)$ (1) In Equation (1), d_{track} is the predetermined distance between consecutive turning events. So, for example, in the 6MWT and the 2MWT, d_{track} is 30 meters, and in the TUG walking test, d_{track} is 3 meters. In addition, "test start time" is the time of day the subject begins the walking test, and "time of last turn" is the time of day the subject executes the last turning event of the one or more turning events in the test-specific pattern. The reference walking speed for the walking test is the average speed.

In another embodiment, the average walking speed is determined for each discrete segment between each set of consecutive turning events during the walking test, which may be calculated according to Equation (2), such that N average speeds will be calculated for N segments: $Average Speed = {d}_{track} / time between two turns$ (2) In Equation (2), d_{track} is the predetermined distance of the segment between consecutive turning events. So, in the 6MWT and the 2MWT, d_{track} is 30 meters, and in the TUG walking test, d_{track} is 3 meters, for example. In addition, "time between two turns" is the elapsed time between two consecutive turning events in the test-specific pattern performed by the subject. In this embodiment, the reference walking speed for the entire walking test may be the average of the average speeds determined from the N discrete segments. Further, additional calibration factors may be determined using the average speeds from the N discrete segments, such as median (to eliminate outliers) and standard deviation (to predict confidence intervals of future speed predictions), for example.

In another embodiment, the average speed is determined, e.g., using the TUG walking test, based on cumulative distance walked by the subject between all of the turning events over the entire time to perform the walking test, which may be calculated according to Equation (3): $Average Speed = \left(number of turns*2{d}_{track}\right) / \left(total walking time\right)$ (3)

In Equation (3), d_{track} is the predetermined distance of a segment between consecutive turning events. So, in the 6MWT and the 2MWT, 2d_{track} is 60 meters, and in the TUG walking test, 2d_{track} is 6 meters, for example. Also, the "total walking time" is the elapsed time between the start of the walking test (test start time) and the end of walking test (test end time). When the walking test begins and ends with the subject standing up and sitting down, e.g., as in the TUG walking test, the "total walking time" may be determined according to Equation (4) to account for the subject's movement and/or change in posture at the start and end of the walking test. This includes the time it takes to initially transition from a sitting position to a standing position at the start of the walking test and to finally transition from the standing position to the sitting position at the end of the walking test: $\begin{matrix}Total walking time = \left(test end time-final posture change duration\right) - \\ \left(test start time-initial posture change duration\right)\end{matrix}$ (4)

The "initial posture change duration" is the time it takes for the subject to stand up and begin walking at the start of the walking test, and the "final posture change duration" is the time it takes for the user to stop walking, turn around and sit down at the end of the walking test.

In a related embodiment, Equation (3) may be split into two average speed calculations, according to Equations (5A) and (5B): $First Average Speed = {d}_{track} / walk to turn time$ (5A) $Second Average Speed = {d}_{track} / turn to stop time$ (5B)

**In** this case, either the first or the second average speed may be used as the reference walking speed, or some combination (e.g., average) of the first and second average speeds may be used as the reference walking speed. Alternatively, correction coefficients may be derived based on the first and second average speeds, respectively. These two correction coefficients should agree with each other or otherwise be rejected, thereby providing some quality indication that the calibration procedure is successful.

In Equation (5A), the "walk to turn time" is the time it takes for the subject to walk from the starting position to the turn at the end of the predetermined distance (d_{track}). The "walk to turn time" may be determined according to Equation (6A) to include the time taken for the subject's initial movement and/or change in position at the start of the walking test when transitioning from the sitting position to the standing position: $Walk to turn time = turn time - \left(test start time-initial posture change duration\right)$ (6A)

In Equation (5B), the "turn to stop time" is the time it takes for the subject to walk from the turn to the starting position across the predetermined distance (d_{track}). The "turn to stop time" may be determined according to Equation (6B) to include the time taken for the subject's final movement and/or change in position at the end of the walking test when transitioning from the standing position to the sitting position: $Turn to stop time = \left(test end time-final posture change duration\right) - turn time$ (6B)

Referring again to FIG. 2, in block S212, a calibration estimated walking speed of the subject is predicted using a predetermined walking speed algorithm, mentioned above. The calibration estimated walking speed may be based on movement data provided by the one or more sensors worn by the subject during the walking test, as discussed above, such as an accelerometer and/or a gyroscope, for example. The calibration estimated walking speed is predicted during the same walking test during which the reference walking speed is determined based on the turning events.

The walking speed algorithm may be any compatible existing or yet to be determined algorithm within the purview of one of ordinary skill in the art having the benefit of the present disclosure capable of estimating walking speed of a subject based on signals provided by movement sensors, such as accelerometers, gyroscopes, barometers, and/or force sensors, which would be apparent to one skilled in the art. Examples of compatible walking speed algorithms include inverted pendulum model, linear regression algorithms, and machine learning algorithms, such as a feature-based, deep learning neural network, artificial neural network (ANN), or convolutional neural network (CNN), for example. The inverted pendulum model, in particular, is described by Rispens et al., "Validation of Walking Speed Estimation from Trunk Mounted Accelerometers for a Range of Walking Speeds," Sensors, 21:1854 (March 6, 2021), the entire contents of which are hereby incorporated by reference. The machine learning algorithms may be previously trained using annotated training data that associates data from movement sensors with actual walking speeds of subjects (ground truth data) in a training data set.

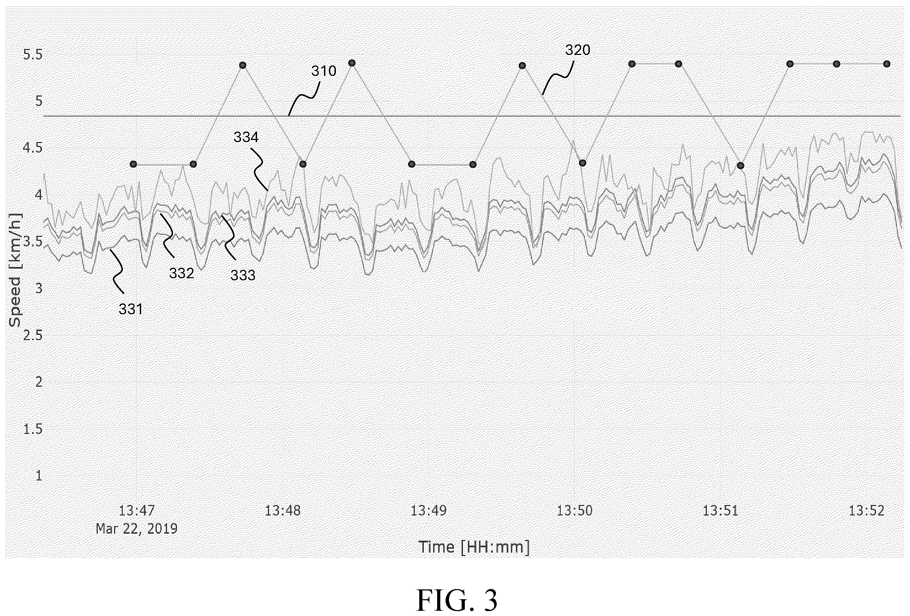

FIG. 3 is a graph showing reference walking speeds and calibration estimated walking speeds, according to a representative embodiment, where the calibration estimated walking speeds are predicted using four walking speed algorithms, respectively. Referring to FIG. 3, trace 310 is a conventionally determined reference walking speed, which is the average walking speed of the subject based on test duration (i.e., 6 minutes) and manually calculated time and distance. This conventionally determined reference walking speed is considered to be the most accurate on average since it is manually calculated. In the depicted example, the conventionally determined average walking speed as shown by trace 310 is 4.84 km/h.

Trace 320 is reference walking speed determined based on walking pattern variations (e.g., turning events) occurring between turns over the course of the walking test, as discussed above with reference to block S211. Each dot in the trace 320 indicates the time at which a turn occurs and the walking speed of the subject at the end of the segment leading up to the turn. For example, the three dots at the end of the trace 320 indicate the last three turns of the subject during the walking test, each of which occurred at the same walking speed. When determined according to Equation (1), for example, the estimated average walking speed based on walking pattern variations as shown by trace 320 is 4.89 km/h, which is consistent with the conventionally determined average walking speed of 4.84 km/h. Therefore, the average walking speed estimated based on walking pattern variations is sufficiently accurate to be used as the reference walking speed. This average walking speed also provides more points for determining calibration coefficients, as discussed below. For example, part of the turn-to-turn walking data (e.g., from a segment between consecutive turning events) that cannot be used may be excluded, for instance, when the subject stops in the middle of the exercise to catch breath. Excluding part of the data may be done by looking at the distribution of the calibration ratios obtained from each turn-to-turn reference walking speed, and eliminating any calibration coefficient(s) that are outliers as compared to the calibration coefficients for the other ratios.

Traces 331 to 334 are calibration estimated walking speeds of the subject predicted using different walking speed algorithms, respectively, with movement data input from a sensor (e.g., an accelerometer and/or a gyroscope) worn by the subject over the course of a walking test. Each of the walking speed algorithms is able to predict instantaneous walking speeds at discrete times, thereby generating the corresponding traces 331 to 334. In the depicted example, trace 331 shows the calibration estimated walking speed of the subject as predicted by an inverted pendulum model, for example, discussed above. The remaining traces 332, 333 and 334 show the calibration walking estimated speeds of the subject as predicted by known linear regression algorithms with different coefficients and features extracted from the accelerometer signals, such as average amplitude and standard deviation of acceleration in the last six seconds, for example, discussed above. Notably, the dips in the traces 331 to 334 correspond to turning events that may be detected by the turn detection algorithm, as discussed above. Alternatively, the calibration estimated walking speeds of the subject may be predicted by existing or yet to be determined deep learning algorithms (end-to-end models) within the purview of one of ordinary skill in the art having the benefit of the present disclosure that input acceleration signals and output walking speeds predicted based on the input acceleration signals, discussed above. Of course, other walking speed algorithms or models may be incorporated to obtain the calibration estimated walking speeds without departing from the scope of the present teachings, as would be apparent to one skilled in the art. As shown in FIG. 3, all of the walking speed algorithms significantly underestimate the subject's walking speed over the course of the walking test. Therefore, calibration coefficients are calculated in order to correct for the errors of the respective walking speed algorithms.

Accordingly, in block S213 of FIG. 2, at least one calibration coefficient is determined for the subject based on the reference walking speed from block S211 and the calibration estimated walking speed from block S212. In an embodiment, the calibration coefficient is determined as a ratio between the reference walking speed determined based on the turning events and the calibration estimated walking speed predicted using an existing or yet to be determined walking speed algorithm within the purview of one of ordinary skill in the art having the benefit of the present disclosure. Notably, the calibration coefficient is specific to the subject and the particular walking speed algorithm used to determine the calibration estimated walking speed. In an embodiment, the calibration coefficient may be updated over time based on recovery time of the subject and/or performance of a different standardized walking test by the subject.

In an embodiment, multiple calibration coefficients may be calculated for one walking test whenever the walking test determines different average walking speeds for different parts of the walking test. For example, referring Equation (2) above, a calibration coefficient may be calculated for each discrete segment of the walking test (segment calibration coefficient), where each discrete segment is between consecutive turning events. Accordingly, the final calibration coefficient may be determined based on some combination of the segment calibration coefficients for the different segments. For example, the final calibration coefficient may be the median of the segment calibration coefficients. Or, the final calibration coefficient may be determined by removing at least one outlier of the segment calibration coefficients, and then averaging the remaining segment calibration coefficients to obtain the final calibration coefficient.

In an embodiment, when the walking test includes multiple turning events, the turning events are identified using the at least one sensor, and multiple segments of the walking test between consecutive turning events of the identified multiple turning events are likewise identified. **In** this case, the reference walking speed of the subject may be estimated in block S211 by estimating reference walking speeds for the multiple segments, respectively. Likewise, the calibration estimated walking speed of the subject may predicted in block S212 by predicting the calibration estimated walking speeds for the multiple segments, respectively. In this case, the at least one calibration coefficient may be determined in block S213 as a multiplicative coefficient obtained by linear regression. That is, the multiplicative coefficient is determined by fitting lines between N reference walking speeds for the multiple segments and corresponding N calibration estimated walking speeds for the same segments, respectively, where N is the number of segments (greater than or equal to 2). The line fittings result in an angular coefficient (i.e., the multiplicative coefficient) plus an offset (intercept).

In an embodiment, a confidence interval may be determined with regard to the calibration coefficient. For example, standard deviation may be calculated for the multiple segment calibration coefficients to determine the confidence interval of the estimated walking speed of the subject during the subsequent walking event. When the standard deviation of the segment calibration coefficients is low, the estimated walking speed has a narrower confidence interval, and vice versa. The confidence interval may be used to express reliability of the calibration coefficient. A narrower confidence interval indicates more confidence that the calibration coefficient and subsequent estimation based on the same is accurate.

Table 1 shows the calibration coefficients (α) calculated for the walking speed algorithms shown in FIG. 3 (first to fourth algorithms), respectively, where each of the calibration coefficients (α) is a ratio of the reference walking speed based on turning events (Turns Ref. Speed) and the calibration estimated walking speed predicted by each of the walking speed algorithms.

**Table 1**

| Metric | Ref. Speed | Turns Ref. Speed | First Algorithm | Second Algorithm | Third Algorithm | Fourth Algorithm |
|---|---|---|---|---|---|---|
| Ave. Speed (km/h) | 4.84 | 4.89 | 3.53 | 3.76 | 3.83 | 4.06 |
| Error (km/h) | 0 | 0.05 | -1.31 | -1.08 | -1.01 | -0.78 |
| Error (%) | 0 | 1 | -27 | -22 | -21 | -16 |
| α | - | - | 1.39 | 1.30 | 1.28 | 1.20 |

From the Table 1, it can be seen that the reference walking speed based on turning events provides an accurate average speed estimation, as discussed above, having only a one percent error as compared to the conventionally determined reference walking speed. Also, the walking speed algorithms significantly underestimate the reference walking speeds. That is, the first algorithm is 1.31 km/h less than the reference walking speed (-27 percent error); the second algorithm is 1.08 km/h less than the reference walking speed (-22 percent error); the third algorithm is 1.01 km/h less than the reference walking speed (-21 percent error); and the fourth algorithm is 0.78 km/h less than the reference walking speed (-16 percent error). Using the ratios of the reference walking speed to the calibration estimated walking speeds, the calibration coefficients (α) calculated as 1.39, 1.30, 1.28 and 1.20, respectively.

Referring again to FIG. 2, in block S214, a subsequent estimated walking speed of the subject is predicted during a subsequent walking event, which takes place after the walking test used to determine the calibration coefficient. The subsequent estimated walking speed is determined using the same walking speed algorithm used to predict the calibration estimated walking speed of the subject during the walking test in block S212 and to calculate the calibration coefficient in block S213. The subsequent walking event does not need to follow a specific walking test since the walking speed is determined by the walking speed algorithm, the error of which is corrected using the calibration coefficient. Types of subsequent walking events may include free-living condition walking, treadmill walking, or standardized walking tests, for example.

In block S215, a corrected walking speed of the subject is determined during the subsequent walking event by adjusting the subsequent estimated walking speed using the at least one calibration coefficient. When the calibration coefficient is the ratio between of the reference walking speed based on turning events and the calibration estimated walking speed, as described above, the corrected walking speed is determined by multiplying the subsequent estimated walking speed by the ratio. When the calibration coefficient is a multiplicative coefficient, the corrected walking speed of the subject is determined by multiplying the subsequent estimated walking speed by the multiplicative coefficient, and adding the offset to the product. The corrected walking speed is output to the subject and/or to an observer (e.g., doctor, nurse, physical therapist, or other clinician) in order to inform them of the accurate walking speed.

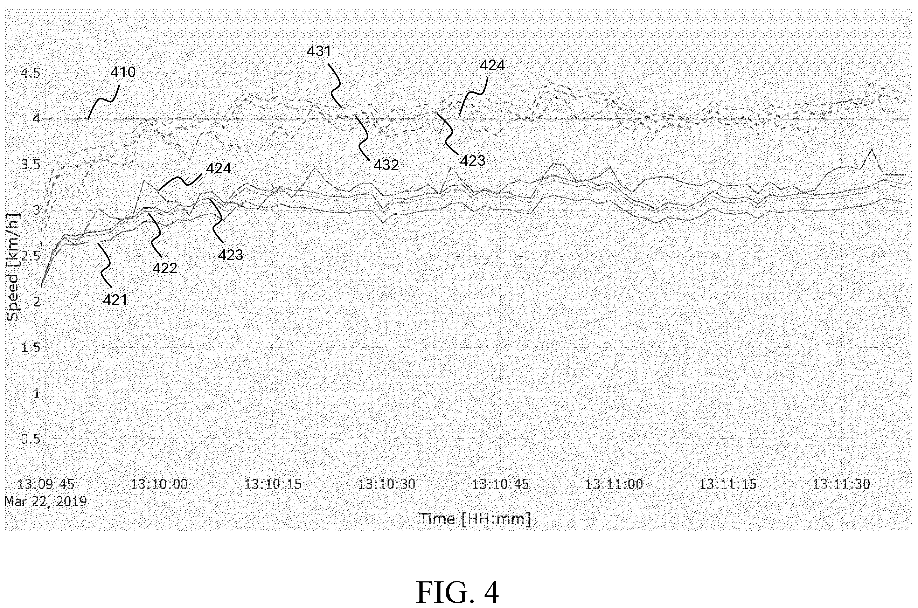

FIG. 4 is a graph showing a known walking speed, subsequent estimated walking speeds predicted by the four different walking speed algorithms, and corrected walking speeds determined during a subsequent walking event, according to a representative embodiment. Referring to FIG. 4, trace 410 is a known walking speed of 4 km/h, which may be set and maintained using a treadmill, for example, to provide the subsequent walking event. In the depicted example, the subsequent walking event lasts for two minutes.

Traces 421 to 424 are subsequent estimated walking speeds of the subject predicted using the different walking speed algorithms, respectively, with movement data input from a sensor (e.g., an accelerometer and/or a gyroscope) worn by the subject over the course of the walking event. The walking speed algorithms used to generate traces 421 to 424 are the same as those used to generate traces 331 to 334 in FIG. 3, respectively, as discussed above. That is, in the depicted example, trace 421 shows the subsequent estimated walking speed of the subject as predicted by an inverted pendulum model, for example, discussed above. The remaining traces 422, 423 and 424 show the subsequent estimated walking speeds of the subject as predicted by known linear regression algorithms with different coefficients and features extracted from the accelerometer signals, for example, discussed above. Alternatively, the calibration estimated walking speeds of the subject may be predicted by deep learning algorithms that input acceleration signals and output walking speeds predicted based on the input acceleration signals, discussed above. Of course, existing or yet to be determined walking speed algorithms within the purview of one of ordinary skill in the art having the benefit of the present disclosure may be incorporated to obtain the subsequent estimated walking speed without departing from the scope of the present teachings, as would be apparent to one skilled in the art.

Like FIG. 3, all of the walking speed algorithms shown in FIG. 4 significantly underestimate the subject's walking speed over the course of the walking event, and are therefore corrected using the calibration coefficients (α) corresponding to the different walking speed algorithms, respectively. That is, in the depicted example, trace 431 shows the corrected walking speed of the subject from trace 421, trace 432 shows the corrected walking speed of the subject from trace 422, trace 433 shows the corrected walking speed of the subject from trace 423, and trace 434 shows the corrected walking speed of the subject from trace 424. As can be seen, each of the corrected traces 431 to 434 substantially corresponds to the reference walking speed trace 410, verifying the accuracy of the calibration coefficients (α).

Table 2 shows the calibration coefficients (α) calculated for the walking speed algorithms shown in FIG. 4, where each of the calibration coefficients (α) is the ratio of the reference walking speed based on turning events (Turns Ref. Speed) and the calibration estimated walking speed predicted by each of the walking speed algorithms, as discussed above.

**Table 2**

| Metric | True Speed | First Algorithm | Second Algorithm | Third Algorithm | Fourth Algorithm |
|---|---|---|---|---|---|
| Ave. Speed (km/h) | 4.00 | 2.89 | 3.01 | 3.05 | 3.13 |
| Error (km/h) | - | -1.11 | -0.99 | -0.95 | -0.87 |

| After Applying Calibration Coefficients (α) | | | | | |
|---|---|---|---|---|---|
| α | | 1.39 | 1.30 | 1.28 | 1.20 |
| Ave. Speed (km/h) | 4.00 | 4.01 | 3.91 | 3.90 | 3.77 |
| Error (km/h) | - | 0.01 | -0.09 | -0.10 | -0.23 |
| Improvement (%) | - | 28 | 23 | 21 | 16 |

Table 2 shows significant improvement of the estimated average walking speeds predicted by the walking speed algorithms after adjusting them using the corresponding calibration coefficients (α). That is, the error introduced by the first algorithm is reduced from -1.11 km/h to 0.01 km/h (28 percent improvement); the error introduced by the second algorithm is reduced from -0.99 km/h to -0.09 km/h (23 percent improvement); the error introduced by the third algorithm is reduced from 1-0.95 km/h to -0.10 km/h (21 percent improvement); and the error introduced by the fourth algorithm is reduced from -0.87 km/h to -0.23 km/h (16 percent improvement). Accordingly, it is shown that all of the walking algorithms significantly improve accuracy when offset by the calibration coefficient (α).

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to serve as a complete description of all of the elements and features of the disclosure described herein. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

## Claims

1. A method of monitoring walking speed of a subject, the method comprising:
estimating a reference walking speed of the subject based on walking pattern variations in a test-specific pattern of a walking test over a known distance;
predicting a calibration estimated walking speed of the subject using a predetermined walking speed algorithm based on data from at least one sensor worn by the subject during the walking test;
determining at least one calibration coefficient based on the reference walking speed and the calibration estimated walking speed;
predicting a subsequent estimated walking speed of the subject during a subsequent walking event using the predetermined walking speed algorithm after the walking test; and
determining a corrected walking speed of the subject during the subsequent walking event by adjusting the subsequent estimated walking speed using the at least one calibration coefficient.

2. The method of claim 1, where the walking pattern variations comprise one or more of a turning event, a body position transition, a change in body posture of the subject, or starting and stopping walking.

3. The method of claims 1 or 2, wherein the at least one calibration coefficient is determined based on as a ratio between the reference walking speed and the calibration estimated walking speed.

4. The method of any of the preceding claims, wherein the walking test comprises a standardized six-minute walking test (6MWT), a standardized two-minute walking test (2MWT), or a standardized timed up and go (TUG) walking test.

5. The method of any of the preceding claims, wherein estimating the reference walking speed of the subject based on walking pattern variations comprises identifying a plurality of turning events during the walking test using the at least one sensor, and identifying a plurality of segments between consecutive turning events of the plurality of turning events;
wherein estimating the reference walking speed of the subject comprises estimating reference walking speeds for the plurality of segments, respectively;
wherein predicting the calibration estimated walking speed of the subject comprises predicting the calibration estimated walking speeds for the plurality of segments, respectively; and
wherein the at least one calibration coefficient is a multiplicative coefficient, and wherein determining the at least one calibration coefficient comprises fitting lines between the calibration walking speeds and the reference walking speeds for of the plurality of segments, respectively, to obtain an angular coefficient and an offset, wherein the angular coefficient is the multiplicative coefficient.

6. The method of claim 5, wherein determining the corrected walking speed of the subject during the subsequent walking event comprises adjusting the subsequent estimated walking speed using the multiplicative coefficient and the offset.

7. The method of claims 1-4, wherein estimating the reference walking speed of the subject based on walking pattern variations comprises:
identifying at least one turning event during the walking test using the at least one sensor; and
computing average speed as the reference walking speed as follows, wherein d_{track} is a predetermined distance between turns: $Average Speed = \left(number of turns*{d}_{track}\right) / \left(time of last turn-test start time\right) .$

8. The method of claims 1-4, wherein estimating the reference walking speed of the subject based on walking pattern variations comprises:
identifying at least one turning event during the walking test using the at least one sensor, wherein d_{track} is a predetermined distance between adjacent turning events; and
computing average speed for at least one every discrete segment between the adjacent turning events as the reference walking speed as follows: $Average Speed = {d}_{track} / time between two turns .$

9. The method of claim 8, wherein the calibration estimated walking speed of the subject is predicted between the turning events using the predetermined walking speed algorithm; and
wherein determining at least one calibration coefficient comprises calculating segment calibration coefficients as ratios between the reference walking speed and each of the calibration estimated walking speeds, and determining the at least one calibration coefficient based on the segment calibration coefficients.

10. The method of claim 9, wherein the at least one calibration coefficient is the median of the segment calibration coefficients.

11. The method of claim 9, wherein determining the at least one calibration coefficient further comprises removing at least one outlier of the segment calibration coefficients, and wherein the at least one calibration coefficient is the average of remaining segment calibration coefficients.

12. The method of claim 9, further comprising:
using standard deviation in the calculated segment calibration coefficients to calculate a confidence interval of the subsequent estimated walking speed of the subject during the subsequent walking event, wherein when the standard deviation of the segment calibration coefficients is low, the subsequent estimated walking speed has a narrower confidence interval.

13. A system for monitoring walking speed of a subject, the system comprising:
at least one sensor worn by the subject;
at least one processor configured to receive data from the at least one sensor; and
at least one non-transitory memory storing instructions that, when executed by the at least one processor to, cause the at least one processor to:
estimate a reference walking speed of the subject based on walking pattern variations in a test-specific pattern of a walking test over a known distance;
predict a calibration estimated walking speed of the subject using a predetermined walking speed algorithm based on data from at least one sensor during the walking test;
determine at least one calibration coefficient based on the reference walking speed and the calibration estimated walking speed;
predict a subsequent estimated walking speed of the subject during a subsequent walking event using the predetermined walking speed algorithm after the walking test; and
determine a corrected walking speed of the subject during the subsequent walking event by adjusting the subsequent estimated walking speed using the at least one calibration coefficient.

14. The system of claim 13, wherein the at least one sensor comprises at least one of an accelerometer, a gyroscope, a barometer, or a force sensor.

15. A computer program product comprising instructions to cause the system of claims 13 or 14 to execute the steps of the method of claims 1-12.
